Europäisches Patentamt

European Patent Office

Office européen des brevets

(19)

(11) **EP 0 529 057 B1**

(12) **EUROPEAN PATENT SPECIFICATION**

(45) Date of publication and mention
of the grant of the patent:
**16.07.1997 Bulletin 1997/29**

(21) Application number: **92908459.8**

(22) Date of filing: **05.03.1992**

(51) Int Cl.[6]: **G01N 33/53**, G01N 33/543,
G01N 33/569, G01N 33/576

(86) International application number:
**PCT/US92/01611**

(87) International publication number:
**WO 92/15881 (17.09.1992 Gazette 1992/24)**

(54) **DENATURED VEHICULAR PROTEINS TO IMPROVE ENZYME LINKED IMMUNOSORBENT ASSAYS**

DENATURIERTE TRANSPORTPROTEINE ZUR VERBESSERUNG VON ENZYMGEBUNDENEN IMMUNOASSAYS (ELISA)

PROTEINES VEHICULAIRES DENATUREES DESTINEES A AMELIORER LES ESSAIS PAR IMMUNOSORBANT LIE A UNE ENZYME

(84) Designated Contracting States:
**AT BE CH DE DK ES FR GB GR IT LI LU MC NL SE**

(30) Priority: **06.03.1991 US 665036**

(43) Date of publication of application:
**03.03.1993 Bulletin 1993/09**

(73) Proprietors:
• **ORTHO DIAGNOSTIC SYSTEMS INC.
Raritan, New Jersey 08869-0602 (US)**
• **CHIRON CORPORATION
Emeryville, California 94608 (US)**

(72) Inventors:
• **BAHL, Chander, P.
Flemington, NJ 08822 (US)**
• **BATSKO, Eleanor
Chester, NJ 07930 (US)**
• **NELLES, Mitchell
Succasunna, NJ 07876 (US)**
• **GARCIA, Gerard
Washington, NJ (US)**
• **CHIEN, David
Alamo, CA 94507 (US)**

(74) Representative: **VOSSIUS & PARTNER
Postfach 86 07 67
81634 München (DE)**

(56) References cited:
WO-A-84/04395            US-A- 4 256 724
US-A- 4 407 943          US-A- 4 658 022
US-A- 4 680 274          US-A- 4 745 182
US-A- 4 820 642          US-A- 4 904 581
US-A- 4 959 323          US-A- 5 106 726

• **Canadian Journal of Microbiology, Volume 36, issue 9, issued September 1990, G. H. BOWDEN et al., "Study of the Antigenic Relationship between Strains of Bacteroides. Indermedius, B. Melaninogenicus, B. Corporis, and B. Denticola Revealed by Immunoblotting with Rabbit Antisera", pages 637-648.**
• **ED HARLOW and DAVID LANE, "Antibodies a Laboratory Manual", published 1988 by Cold Spring Harbor Laboratory (New York), see pages 100-101, 124, 127, 632, 633.**
• **P. TIJSSEN, "Practice and Theory of Enzyme Immunoassays", published 1985 by Elsevier (New York), see pages 9-20, 373-375 and 479-581.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

## BACKGROUND OF THE INVENTION

### Field of the Invention

This invention pertains to enzyme linked immunosorbent assays having improved specificity. Specifically, the invention pertains to assays wherein the binding of a test sample to the vehicular protein in a recombinant fusion protein is suppressed by premixing the sample with the vehicular protein in denatured form. The improved method of the present invention may be employed in a wide variety of enzyme linked immunosorbent assays to analyze a wide variety of substances.

### Description of the Background

Numerous types of enzyme linked immunosorbent assays (ELISA) are known for the analysis of substances such as antigens and antibodies in biological fluids. In an enzyme linked immunosorbent assay, the concentration of the analysandum (the substance to be analyzed) is determined by binding the analysandum with a reactant which is a protein having a specific binding affinity for the analysandum. The concentration of the analysandum is measured by labeling with an enzyme the reactant which has a specific binding affinity for the analysandum or a quantity of a reactant which is the same as the analysandum. Enzyme labels are generally very sensitive labels because enzymes can amplify weak concentration signals through catalysis reactions.

Although immunoassays may be utilized to analyze for both antigens and antibodies, immunoassays will be described hereafter in terms of analyzing for antigens. Enzyme linked immunosorbent assays are classified as either homogeneous assays or heterogeneous assays. In a homogeneous assay, the enzyme activity of the assay solution can be measured without separating the antibody bound antigens from the unbound antigens because the enzyme activity of the antibody bound antigens is significantly different from that of the unbound antigens. In a heterogeneous assay, the enzyme activity of the assay solution is measured after the antibody bound antigens are separated, generally by immobilization, from the unbound antigens. In general, a homogeneous assay is easier to conduct and to automate but a heterogeneous assay is more sensitive.

In a homogeneous enzyme linked immunosorbent assay, a known amount of enzyme-labeled antigen competes with test sample antigen for a known limited amount of antibody to form an enzyme-labeled antigen/antibody complex. The presence of the antibody in the enzyme-labeled antigen/antibody complex causes the complex to have very little enzyme activity because of steric hindrance or allosteric inhibition. The enzyme activity of the assay solution is directly proportional to the amount of test sample antigen. A competitive homogeneous enzyme linked immunosorbent assay is schematically set out below, wherein An is an antigen, Ab is an antibody, and Ez is an enzyme.

$$
\underset{\substack{(known) \\ (active)}}{An\text{-}Ez} \quad + \quad \underset{(unknown)}{An} \quad \xrightarrow{\ \ \overset{Ab}{|}\ (limited)\ \ } \quad \underset{(inactive)}{\overset{\overset{\displaystyle Ab}{|}}{An\text{-}Ez}} \quad + \quad \overset{\overset{\displaystyle Ab}{|}}{An}
$$

The precision and accuracy of homogeneous enzyme linked immunosorbent assays are comparable to those of other immunological methods, such as radioimmunoassay, and other non-immunological methods, such as gas chromatography, high pressure liquid chromatography, and thin layer chromatography. Other types of homogeneous enzyme linked immunosorbent assays are known such as assays using enzyme modulators, assays using enzyme prosthetic groups, assays using fluorogenic enzyme substrates, assays based on antibody induced restriction on the conformation of apoenzyme labeled ligand, assays using enzyme channeling, assays using liposome-entrapped enzymes, and assays using reagent strip format.

The principles and procedures of heterogeneous enzyme linked immunosorbent assays are essentially the same as those for radioimmunoassays. For example, in a competitive heterogeneous enzyme linked immunosorbent assay, a known amount of soluble enzyme-labeled antigen competes with test sample antigen for a known limited amount of immobilized antibody. After reaction, the immobilized antibody phase containing the bound enzyme-labeled antigen is separated from the soluble phase. The enzyme activity of the enzyme-labeled antigen/immobilized antibody phase is inversely proportional to the amount of test sample antigen. A competitive heterogeneous enzyme linked immunosorbent assay is schematically set out below, wherein An is an antigen, Ab is an antibody, Ez is an enzyme, and phase is an immobilized phase.

```
                              Ab
                              |            An-Ez         An
                              |            |             |
                              phase        |             |
       An-Ez     +     An     ————————>    Ab      +     Ab
                              (limited)    |             |
       (known)         (unknown)           phase         phase
```

In an inhibition heterogeneous enzyme linked immunosorbent assay, a known amount of immobilized antigen competes with test sample antigen for a known limited amount of soluble enzyme-labeled antibody. After reaction, the immobilized antigen phase containing the bound enzyme-labeled antibody is separated from the soluble phase. The enzyme activity of the enzyme-labeled antibody/immobilized antigen phase is inversely proportional to the amount of test sample antigen.

```
                              Ab
                              |            Ab-Ez         Ab-Ez
                              Ez           |             |
       An        +     An     ————————>    An      +     An
       |                      (limited)    |
       phase                               phase

       (known)         (unknown)
```

Sandwich heterogeneous enzyme linked immunosorbent assays are used to analyze for antigens which have multiple epitopes (the site or determinant on an antigen which attaches to an antibody) or antibodies which have multiple paratopes (the site on an antibody which attaches to an antigen). In a direct sandwich assay, a test sample antigen, which has multiple epitopes, is reacted with an excess of immobilized antibody-1. After reaction and separation of the immobilized antibody-1 phase containing the bound antigen, an excess or known amount of enzyme-labeled antibody-2 is added to the immobilized phase to further react with the antigen. The enzyme activity of the enzyme-labeled antibody-2/antigen/immobilized antibody-1 phase is directly proportional to the amount of test sample antigen.

```
       An        +     $Ab_1$      ————————>    $Ab_1$-An
                       |                        |
                       phase                    phase

       (unknown)       (excess)
```

```
                       $Ab_2$
                       |
                       Ez
                       ————————>    $Ab_1$-An-$Ab_2$-Ez
                       (known)      |
                                    phase
```

Indirect sandwich heterogeneous enzyme linked immunosorbent assays are used to measure serum levels of specific antibodies produced in response to a pathogen. In an indirect sandwich assay, a test sample antibody-1, which has multiple paratopes, is reacted with an excess of immobilized antigen. After reaction and separation of the immobilized antigen phase containing the bound antibody-1, an excess or known amount of enzyme-labeled antispecies immunoglobulin antibody-2 is added to the immobilized phase to further react with antibody-1. The enzyme activity of the enzyme-labeled antibody-2/antibody-1/immobilized antigen phase is directly proportional to the amount of antibody in the test sample.

$$An \mid phase \quad + \quad Ab_1 \quad \longrightarrow \quad An\text{-}Ab_1 \mid phase$$

$$(excess) \quad (unknown)$$

$$\begin{array}{c} Ab_2 \\ \mid \\ Ez \\ \longrightarrow \\ (known) \end{array} \quad \begin{array}{c} An\text{-}Ab_1\text{-}Ab_2\text{-}Ez \\ \mid \\ phase \end{array}$$

In a sandwich assay, the first immunochemical reaction can be carried out in a volume larger than is desirable for the second reaction such as when the analysandum is present in a low concentration. In addition, the biological fluid in the first reaction may contain substances which would adversely effect the second immunochemical reaction, the enzyme present in the conjugate, or the subsequent enzymatic assay reaction.

Other types of heterogeneous enzyme linked immunosorbent assays are known such as immunoenzymometric assays, tagged enzyme ligand conjugate assays, steric hindrance assays, isoenzyme assays, and amplified enzyme label assays. Enzyme linked immunosorbent assays are reviewed in detail in Enzyme Mediated Immunoassays, Ngo and Lenhoff (Editors), "Enzyme Mediated Immunoassay: An Overview", pp. 3-32, Plenum Press, New York, N.Y. (1985).

United States patent no. 3,654,090, Reexamination Certificate issued July 20, 1982, to Schuurs et al., describes a process for determining a component in the antigen-antibody reaction which comprises adding to a test sample an amount of one component in insolubilized form and an adjusted amount of the other component covalently linked to an enzyme. After reaction, the unbound component is separated from the enzyme bound labeled component to form two fractions and the enzyme activity of one fraction is determined which is a measure of the amount of test component in the sample.

United States patent Re. no. 31,006, issued to Schuurs et al., describes a process for the demonstration and determination of a component of the reaction between specific binding proteins. The process comprises reacting the component to be determined with its binding partner in insolubilized form, separating the solid phase of the reaction mixture from the liquid phase, reacting the solid phase with a coupling product obtained by binding a substance capable of reacting specifically with one of the reaction components to an enzyme, and determining the enzyme activity of the liquid or the solid phase of the reaction mixture obtained, which is a measure of the quantity of the substance to be determined.

The concept that many proteins can be made more immunogenic by denaturation is known from "Antibodies A Laboratory Manual", Ed Harlow and David Lane, published 1988 by Cold Spring Harbor Laboratory (New York), pages 100, 101, 124, 127, 632 and 633. Said document also teaches that nonspecific binding in immunoassays can be reduced by adding a source of nonspecific protein.

Thus, many different types of enzyme linked immunosorbent assays are known for analyzing antigens and antibodies in biological fluids. In spite of the sensitivity and specificity that can be achieved by many of these methods, it is still not possible to detect trace amounts of many substances such as antibodies produced in response to a pathogen in the beginning stages of a disease or substances having binding counterparts with low binding affinity. Methods for concentrating the substance to be analyzed are laborious and the results are generally still unsatisfactory. Accordingly, improved assay methods for analyzing low concentrations of antigens and antibodies are desirable. The present invention provides such improved enzyme linked immunosorbent assays for the analysis of antigens and antibodies in high specificity. The improved method of the present invention may be employed in a wide variety of enzyme linked immunosorbent assays to analyze a wide variety of substances.

## SUMMARY OF THE INVENTION

In one embodiment, the present invention pertains to a homogeneous enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;

(b) providing a second reactant which is an enzyme-labeled reactant wherein the enzyme-labeled reactant comprises a reactant which is the same as the analysandum coupled to an enzyme;

(c) preparing a diluent solution of the analysandum by forming a solution of the analysandum with an effective amount of denatured vehicular protein to suppress binding of the analysandum to the vehicular protein in the recombinant fusion protein;

(d) reacting the diluent solution of analysandum from step (c) with the recombinant fusion protein reactant from step (a) and the enzyme-labeled reactant from step (b) to form a reaction mixture, wherein the enzyme-labeled reactant is present in a known amount and the recombinant fusion protein reactant is present in a known amount insufficient to react with the total amount of analysandum and enzyme-labeled reactant; and

(e) analyzing the enzyme activity of the reaction mixture which is directly proportional to the quantity of analysandum present in the reaction mixture.

In another embodiment, the present invention pertains to a heterogeneous enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;

(b) immobilizing the recombinant fusion protein reactant from step (a) by contacting an aqueous solution of the recombinant fusion protein with a solid phase to immobilize the fusion protein on the solid phase;

(c) separating the aqueous solution from the solid phase in step (b) to prepare an immobilized reactant;

(d) providing a second reactant which is an enzyme-labeled reactant wherein the enzyme-labeled reactant comprises a reactant which is the same as the analysandum coupled to an enzyme;

(e) preparing a diluent solution of the analysandum by forming a solution of the analysandum with an effective amount of denatured vehicular protein to suppress binding of the analysandum to the vehicular protein in the recombinant fusion protein;

(f) reacting the diluent solution of analysandum from step (e) with the immobilized recombinant fusion protein reactant from step (c) and the enzyme-labeled reactant from step (d) to form a reaction mixture, wherein the enzyme-labeled reactant is present in a known amount and the recombinant fusion protein reactant is present in a known amount insufficient to react with the total amount of analysandum and enzyme-labeled reactant;

(g) separating the immobilized phase and the liquid phase; and

(h) analyzing the enzyme activity of the immobilized phase which is inversely proportional to the quantity of analysandum present in the reaction mixture.

In yet another embodiment, the present invention pertains to a heterogeneous sandwich enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;

(b) immobilizing the recombinant fusion protein reactant from step (a) by contacting an aqueous solution of the recombinant fusion protein with a solid phase to immobilize the fusion protein on the solid phase;

(c) separating the aqueous solution from the solid phase in step (b) to prepare an immobilized reactant;

(d) preparing a diluent solution of the analysandum by forming a solution of the analysandum with an effective amount of denatured vehicular protein to suppress binding of the analysandum to the vehicular protein in the recombinant fusion protein;

(e) reacting the diluent solution of analysandum from step (d) with the immobilized recombinant fusion protein reactant from step (c) to form a first reaction mixture having a first immobilized phase and a first liquid phase, wherein the immobilized reactant is present in an amount effective to react with all of the analysandum;

(f) separating the first immobilized phase and the first liquid phase in step (e);

(g) providing a second reactant which is an enzyme-labeled reactant wherein the enzyme-labeled reactant comprises a reactant which is a second binding counterpart to the analysandum coupled to an enzyme;

(h) reacting the enzyme-labeled reactant from step (g) with the first immobilized phase from step (f) to form a second reaction mixture having a second immobilized phase and a second liquid phase, wherein the enzyme-labeled reactant is present in a known amount;

(i) separating the second immobilized phase and the second liquid phase in step (h); and

(j) analyzing the enzyme activity of the second immobilized product phase in step (i) which is directly proportional to the quantity of analysandum present in the reaction mixture.

## DETAILED DESCRIPTION OF THE INVENTION

The present invention pertains to enzyme linked immunosorbent assays for analyzing a test sample or analysandum which is a reactant in a reaction of binding counterparts. The binding counterparts comprise a bindable substance such as an antigen or a hapten and a binding substance such as an antibody or a specific binding protein. In the improved assay, a first reactant, which is the binding counterpart of the analysandum, is provided as a recombinant fusion protein. A recombinant fusion protein comprises a first protein having the amino acid sequence and immunological reactivity of the binding counterpart and a second protein which is a vehicular or carrier protein. The first and second proteins are fused to each other. A second reactant is provided which is an enzyme-labeled reactant comprising a reactant which is the same as the analysandum coupled to an enzyme. A diluent solution of the analysandum is prepared by forming a solution of the analysandum with an effective amount of denatured vehicular protein to suppress binding of the analysandum to the vehicular protein in the recombinant fusion protein. The diluent solution of analysandum and the enzyme-labeled second reactant are then competitively reacted with the recombinant fusion protein first reactant to assay for the analysandum.

Applicants have found that a certain amount of non-specific binding often occurs between a test sample and the vehicular protein portion of a recombinant fusion protein to provide a false positive response and thereby decrease the specificity of the assay. Addition of native vehicular protein to the test sample solution as a competitive inhibitor does not suppress this non-specific binding. Applicants have discovered that by adding denatured vehicular protein to the test sample solution, the non-specific binding between the test sample and the vehicular protein portion of the recombinant fusion protein is suppressed. The improved method of the present invention may be used to increase the specificity of a wide variety of enzyme linked immunosorbent assays to analyze a wide variety of substances.

The enzyme linked immunosorbent assays within the scope of the present invention include homogeneous assays and heterogeneous assays. The homogeneous enzyme linked immunosorbent assays may be assays using enzyme modulators, assays using enzyme prosthetic groups, assays using fluorogenic enzyme substrates, assays based on antibody induced restriction on the conformation of apoenzyme labeled ligand, assays using enzyme channeling, assays using liposome-entrapped enzymes, and assays using reagent strip format. The heterogeneous enzyme linked immunosorbent assays may be competitive assays, inhibition assays, direct and indirect sandwich assays, immunoenzymometric assays, tagged enzyme ligand conjugate assays, steric hindrance assays, isoenzyme assays, and amplified enzyme label assays. In a preferred embodiment, the immunoassay is a heterogeneous assay such as a competitive assay, an inhibition assay, a direct sandwich assay, and an indirect sandwich assay. In a more preferred embodiment, the immunoassay is a heterogeneous direct sandwich assay or a heterogeneous indirect sandwich assay. In a most preferred embodiment, the heterogeneous immunoassay is an indirect sandwich assay.

The analysandum or substance to be analyzed in the enzyme linked immunosorbent assay of the present invention is a reactant in a reaction between binding counterparts. The binding counterparts are proteins which have a specific binding affinity for each other. One binding counterpart is a bindable substance which is selected from the group consisting of an antigen and a hapten. The preferred bindable substance is an antigen. The other binding counterpart is a binding substance which is selected from the group consisting of an antibody and a specific binding protein. The preferred binding substance is an antibody.

Antigens are substances which are capable under appropriate conditions of inducing the formation of antibodies and of reacting specifically in some detectable manner with the antibodies so induced. Antigens may be soluble substances, such as toxins and foreign proteins, or particulate substances, such as bacteria or tissue cells. In general, antigens are high molecular weight substances such as simple and conjugated proteins and carbohydrates.

The bindable substance may also be a low molecular weight substance such as a hapten. Haptens are specific protein-free substances which have a chemical configuration which can interact with specific binding groups on an antibody but which, unlike antigenic determinants, does not itself elicit the formation of a detectable amount of antibody. When haptens are coupled with a carrier protein to form a conjugate, the hapten can elicit an immune response. In humoral immunity, antibody specificity is directed primarily at the hapten. In cell mediated immunity, antibody specificity is directed at both the hapten and the carrier protein.

The bindable substances in the present invention are substances from natural sources or may be substances prepared by synthetic or recombinant means. In a preferred embodiment, the bindable substance is an antigen selected from the group consisting of recombinant proteins and synthetic peptides. In a more preferred embodiment, the antigen is selected from the group consisting of hepatitus C virus (HCV) and human immunedeficiency virus (HIV).

Antibodies are immunoglobulin molecules which have a specific amino acid sequence which permit it to interact only with the antigen which induced its synthesis in lymphoid tissue or with an antigen closely related to that antigen. Immunoglobulins are proteins made up of two light chains and two heavy chains.

The binding substance may also be a specific binding protein such as an unattached receptor protein or a transport protein. Receptor proteins include proteins which remain attached to cells such as antibodies and unattached proteins which are released to blood serum and retain their specific binding affinity. Transport proteins are proteins that move substances in and out of cells and across epithelial layers in biological systems.

The binding substances may be substances from natural sources or may be substances prepared by synthetic or recombinant means. In a preferred embodiment, the bindable substance is an antibody selected from the group consisting of recombinant proteins and synthetic peptides. In a more preferred embodiment, the antibody is selected from the group consisting of hepatitus C virus antibody and human immunedeficiency virus antibody.

The medium in which the analysandum occurs may be any biological fluid such as serum, plasma, urine, cell culture medium, or synovial fluid. The analysandum may be analyzed directly in the biological fluid, may be extracted from the fluid and concentrated, or may be diluted to decrease the influence of competing reactions.

In general, the pH value of the biological fluid or test sample may be adjusted to a pH value to optimize the immunochemical reaction. For example, the pH value of the test sample may be adjusted to a value from about 5 to about 9.5, preferably from about 8.5 to about 9.5, and more preferably about 9. The pH value of the test sample may be adjusted by adding to the fluid a small amount of a concentrated buffer solution or a dry buffer salt. Typical buffer systems include borate buffers, phosphate buffers, citrate buffers, tris(hydroxymethyl)aminomethane buffers, imidazole buffers, and carbonate buffers.

As set out above, the reactant which is the binding counterpart of the test sample in the present invention is provided as a recombinant fusion protein. The recombinant fusion protein comprises a first protein having the immunological reactivity of the binding counterpart and a second protein which is a vehicular protein fused to the first protein. The vehicular protein in the recombinant fusion protein may be any protein suitable for fusing to the first protein to enhance the yield, stability, and ease of isolation of the recombinant fusion protein and to improve the assay sensitivity. Recombinant fusion proteins and methods for preparing such proteins are discussed in detail in European patent application no. 318,216, published 18 November 1989, and European patent application no. 388,232, published 19 September 1989.

In accord with the present invention, the binding of a test sample to the vehicular protein in a recombinant fusion protein is suppressed by premixing the test sample with a diluent solution of the vehicular protein in denatured form. The vehicular protein in the diluent solution will be the same vehicular protein, or the same type of vehicular protein present, in the recombinant fusion protein. Nonlimiting examples of vehicular proteins include superoxide dismutase, *beta*-galactosidase, and lactamase. In a preferred embodiment, the vehicular protein is selected from the group consisting of superoxide dismutase and *beta*-galactosidase, and in a more preferred embodiment, the vehicular protein is superoxide dismutase.

The amount of vehicular protein in the diluent solution is an effective amount to suppress binding of the analysandum to the vehicular protein in the recombinant fusion protein. An effective amount of vehicular protein is a matter of preference subject to such factors as the type and binding affinity of vehicular protein employed, the amount and type of test sample being assayed, and the level of specificity desired. Thus, the exact amount of vehicular protein may be varied in order to obtain the result desired in the final product and such variations are within the capabilities of those skilled in the art without the need for undue experimentation. In a preferred embodiment, the vehicular protein will be present in the diluent solution in an amount from about 30μg/ml to about 300μg/ml, preferably from about 100μg/ml to about 200μg/ml, and more preferably about 100μg/ml, by weight of the diluent solution.

In general, the pH value of the diluent solution will be adjusted to optimize the immunochemical reaction. For example, the pH value of the diluent solution may be adjusted to a value from about 5 to about 9.5, preferably from about 6 to about 8, and more preferably about 7.3. Typical buffer systems which can be employed to adjust the pH value of the diluent solution have been described above.

The diluent solution also may be formulated with conventional ingredients which offer a variety of properties to suit particular applications. Without being limited thereto, such ingredients include preservatives, salts to increase the ionic strength of the solution, and various proteins to reduce non-specific binding of the test sample such as detergents, yeast extract, casein, and bovine serum albumin.

The vehicular protein in the diluent solution may be denatured in any conventional manner. For example, the vehicular protein may be denatured using denaturing agents (e.g., chaotropic agents) or may be denatured with reduction, heat, or with a detergent. Methods for denaturing the vehicular proteins are well known in the art.

Once prepared, the premix of test sample and diluent solution of denatured vehicular protein may be employed in any homogeneous or heterogeneous enzyme linked immunosorbent assay to analyze a wide variety of substances.

In a homogeneous enzyme linked immunosorbent assay, the premixture of test sample in the diluent solution competes with a known amount of enzyme-labeled reactant, which is the same as the test sample, for a known limited amount of binding counterpart, which is provided as a recombinant fusion protein. In a specific embodiment, the invention is directed at an enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;

(b) providing a second reactant which is an enzyme-labeled reactant wherein the enzyme-labeled reactant comprises a reactant which is the same as the analysandum coupled to an enzyme;

(c) preparing a diluent solution of the analysandum by forming a solution of the analysandum with an effective amount of denatured vehicular protein to suppress binding of the analysandum to the vehicular protein in the recombinant fusion protein;

(d) reacting the diluent solution of analysandum from step (c) with the recombinant fusion protein reactant from step (a) and the enzyme-labeled reactant from step (b) to form a reaction mixture, wherein the enzyme-labeled reactant is present in a known amount and the recombinant fusion protein reactant is present in a known amount insufficient to react with the total amount of analysandum and enzyme-labeled reactant; and

(e) analyzing the enzyme activity of the reaction mixture which is directly proportional to the quantity of analysandum present in the reaction mixture.

In a heterogeneous enzyme linked immunosorbent assay, the premixture of test sample in the diluent solution reacts with an immobilized reactant, which is provided as a recombinant fusion protein. In a specific embodiment, the invention is directed at enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;

(b) immobilizing the recombinant fusion protein reactant from step (a) by contacting an aqueous solution of the recombinant fusion protein with a solid phase to immobilize the fusion protein on the solid phase;

(c) separating the aqueous solution from the solid phase in step (b) to prepare an immobilized reactant;

(d) providing a second reactant which is an enzyme-labeled reactant wherein the enzyme-labeled reactant comprises a reactant which is the same as the analysandum coupled to an enzyme;

(e) preparing a diluent solution of the analysandum by forming a solution of the analysandum with an effective amount of denatured vehicular protein to suppress binding of the analysandum to the vehicular protein in the recombinant fusion protein;

(f) reacting the diluent solution of analysandum from step (e) with the immobilized recombinant fusion protein reactant from step (c) and the enzyme-labeled reactant from step (d) to form a reaction mixture, wherein the enzyme-labeled reactant is present in a known amount and the recombinant fusion protein reactant is present in a known amount insufficient to react with the total amount of analysandum and enzyme-labeled reactant;

(g) separating the immobilized phase and the liquid phase; and

(h) analyzing the enzyme activity of the immobilized phase which is inversely proportional to the quantity of analysandum present in the reaction mixture.

In a preferred embodiment, the immobilized reactant in the heterogeneous assay is preparing by mixing the reactant with an aqueous solution having an ionic strength value from about 0.05 to about 1.0 and contacting the mixture with a solid phase to immobilize the reactant on the solid phase. The pH value of the reactant solution may be adjusted to optimize the immunochemical reaction.

The ionic strength value of the reactant mixture is that value sufficiently high to minimize ionic binding of the reactant to the solid phase and thereby decrease protein leaching during later stages of the assay. The ionic strength value of the reactant mixture must also be sufficiently low so as not to adversely affect the solubility of the protein or otherwise interfere with the binding of the reactant to the solid phase. The exact ionic strength value is subject to such factors as the type of reactant to be immobilized and the type of solid phase employed to immobilize the reactant. Thus the ionic strength value may be varied in order to obtain the result desired in the final assay and such variations are within the capabilities of those skilled in the art without the need for undue experimentation. In general, the ionic strength value of the mixture of reactant to be immobilized is from about 0.05 to about 1.0, preferably from about 0.1 to about 1, and more preferably from about 0.25 to about 1. Ionic strength values from about 2 to about 5 tend to have a negative effect on the sensitivity of the assay.

Ionic strength is a measure of the average electrostatic interactions among ions in an electrolyte. Ionic strength, $I$, is equal to one-half the sum of the terms obtained by multiplying the molality of each ion by its valence squared, as set out below.

$$I = 1/2 \text{ the sum of } m_i z_i^2$$

wherein $m_i$ is the molality of each ion and $z_i$ is the valence of the ion. For example, the ionic strength of a 1.0 molal solution of sodium chloride [NaCl] is calculated as follows:

$$I = 1/2 \, (1.0)(1)^2 + 1/2 \, (1.0)(1)^2 = 1$$

The ionic strength of a 1.0 molal solution of aluminum sulfate $[Al_2(SO_4)_3]$ is calculated as follows:

$$I = 1/2 \, (2.0) \, (3)^2 + 1/2 \, (3.0) \, (2)^2 = 15$$

The types of salts useful in the present invention are salts which are capable of increasing the ionic strength value of the reactant mixture without interfering with the binding of the reactant to the solid phase or otherwise adversely affecting the sensitivity of the assay. The exact type of salt employed is a matter of preference subject to such factors as the type and concentration of reactant being immobilized and the type of solid phase being employed. Suitable salts that may be employed in the present invention may be selected from the group consisting of alkali metal salts, alkaline earth metal salts, and the like, and mixtures thereof. In a preferred embodiment, the salt may be selected from the group consisting of sodium chloride, potassium chloride, and mixtures thereof. In a more preferred embodiment, the salt is sodium chloride. Salts may be added in solid form or solution form.

The solid phase in which the reactant is bound and immobilized may be any commercially available solid phase generally used for this purpose. Typical solid phases include a microwell as well as solid phase particles in a tube or container. In general, the solid phase will be made of a material which will bind and immobilize the reactant by a combination of ionic and hydrophobic interaction forces. Suitable materials for solid phases are polymeric materials which have hydrophobic and hydrophilic sites such as those polymers selected from the group consisting of polystyrene, sulfonated polystyrene, irradiated (modified) sulfonated polystyrene, latex, and mixtures thereof. In a preferred embodiment, the solid phase is sulfonated polystyrene.

The aqueous reactant mixture is contacted with the solid phase for a time sufficient for the reactant to bind and be immobilized on the solid phase. In general, the aqueous reactant mixture will be contacted with the solid phase for from about 1 to 6 hours at ambient temperature. After the reactant mixture has been contacted with the solid phase for a sufficient time, the aqueous mixture is separated from the solid phase by any convenient means such as aspiration or decantation to prepare the immobilized reactant. The solid phase may optionally be rinsed or washed with a detergent to remove any non-immobilized or soluble reactant.

The immobilized reactant in the solid phase may optionally be treated with an aqueous solution of an excess of a protein which is a nonreactant in the reaction of binding counterparts. The nonreactant is a protein which will react or saturate any remaining binding or active sites on the solid phase but will not react or interfere with subsequent immunoassay reactions. Treatment of the solid phase with a nonreactant protein helps to prevent non-specific binding of the analysandum to the solid phase. Suitable nonreactant proteins include bovine serum albumen (BSA), gelatin, and ovalvumin. The nonreactant protein will be present in an excess amount to bind all active sites in the solid phase, such as in a concentration of about lmg to 3mg per ml of solution. The aqueous solution of nonreactant is then separated from the solid phase and the solid phase is dried to prepare the immobilized reactant.

In this embodiment, the assay may be a competitive assay or an inhibition assay. In a competitive assay, a reactant which is the binding counterpart of the analysandum is immobilized in a solid phase according to the method of the present invention. An enzyme conjugate is then prepared by coupling an enzyme and a reactant which has the same chemical structure as the analysandum. The analysandum is then mixed with a known quantity of the enzyme conjugate and reacted with a known limited quantity of the immobilized reactant to form a reaction mixture having an immobilized phase and a liquid phase. The analysandum and the enzyme conjugate compete for the limited quantity of immobilized reactant. The known limited quantity of the immobilized reactant corresponds to a quantity which is not sufficient to react with all of the analysandum and the enzyme conjugate. The immobilized phase and liquid phase are then separated. The enzyme activity of the immobilized phase is inversely proportional to the quantity of analysandum.

In an inhibition assay, a reactant which is the same as the analysandum is immobilized in a solid phase according to the method of the present invention. An enzyme conjugate is then prepared by coupling an enzyme and a reactant which is the binding counterpart of the analysandum. The analysandum is then mixed with a known quantity of the immobilized reactant and reacted with a known limited quantity of the enzyme conjugate to form a reaction mixture having an immobilized phase and a liquid phase. The analysandum and the immobilized reactant compete for the limited quantity of enzyme conjugate. The known limited quantity of the enzyme conjugate corresponds to a quantity which is not sufficient to react with all of the analysandum and the immobilized reactant. The immobilized phase and liquid phase are then separated. The enzyme activity of the immobilized phase is inversely proportional to the quantity of analysandum.

The enzyme conjugate is a coupling product of a reactant in the enzyme linked immunosorbent assay and an enzyme. The enzyme may be any enzyme which does not interfere or is not affected by the immunochemical chemical reaction. The exact choice of enzyme is subject to such factors as the ease of the synthesis of the enzyme conjugate, the specific binding activity of the enzyme (a high conversion rate raises the specificity of the assay), and the simplicity of the enzyme assay.

Suitable enzymes which may be use in the enzyme conjugate may be selected from the group consisting of catalase, peroxidases such as horse radish peroxidase (HRP), beta-glucuronidase, beta-B-glucosidase, beta-D-galactosidase, urease, glucose-oxidase, galactose-oxidase, and alkaline phosphatase. In a preferred embodiment, the enzyme may be selected from the group consisting of horse radish peroxidase, beta-D-galactosidase, and alkaline phosphatase. In a more preferred embodiment, the enzyme is horse radish peroxidase.

The preparation of the enzyme conjugate may be carried out by any conventional method subject to such factors as the properties of the particular enzyme and specific binding protein. The enzyme conjugate may be prepared with reagents such as carbodiimides, diisocyanates, glutaric aldehyde, and bis-diazobenzidine. The preparation of the enzyme conjugate should not significantly affect the binding properties of the protein or the activity of the enzyme.

The activity of the enzyme conjugate in the immobilized phase may be measured by reacting the immobilized enzyme conjugate with an enzyme substrate and measuring the conversion rate or activity of the enzyme. Suitable enzyme substrates include 1,2-phenylenediamine (ortho-phenylenediamine), 2,2'-azinobis(3-ethylbenzothiazoline-6-sulfonic acid) diammonium salt (ABTS), and 3,3',5,5'-tetramethylbenzidine (TME). The activity of the enzyme may be measured by any conventional method such as by spectrophotometry, fluorimetry, or colorimetry.

In another embodiment, the invention is directed at a sandwich enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;
(b) immobilizing the recombinant fusion protein reactant from step (a) by contacting an aqueous solution of the recombinant fusion protein with a solid phase to immobilize the fusion protein on the solid phase;
(c) separating the aqueous solution from the solid phase in step (b) to prepare an immobilized reactant;
(d) preparing a diluent solution of the analysandum by forming a solution of the analysandum with an effective amount of denatured vehicular protein to suppress binding of the analysandum to the vehicular protein in the recombinant fusion protein;
(e) reacting the diluent solution of analysandum from step (d) with the immobilized recombinant fusion protein reactant from step (c) to form a first reaction mixture having a first immobilized phase and a first liquid phase, wherein the immobilized reactant is present in an amount sufficient to react with all of the analysandum;
(f) separating the first immobilized phase and the first liquid phase in step (e);
(g) providing a second reactant which is an enzyme-labeled reactant wherein the enzyme-labeled reactant comprises a reactant which is a second binding counterpart to the analysandum coupled to an enzyme;
(h) reacting the enzyme-labeled reactant from step (g) with the first immobilized phase from step (f) to form a second reaction mixture having a second immobilized phase and a second liquid phase, wherein the enzyme-labeled reactant is present in a known amount;
(i) separating the second immobilized phase and the second liquid phase in step (h); and
(j) analyzing the enzyme activity of the second immobilized product phase in step (i) which is directly proportional to the quantity of analysandum present in the reaction mixture.

The sandwich assay may be a direct sandwich assay or an indirect sandwich assay. In a direct sandwich assay, a reactant which is a first antibody to the antigen analysandum, and which is provided as a recombinant fusion protein, is immobilized in a solid phase. The analysandum is then reacted with an excess quantity of the immobilized reactant to form a first reaction mixture having a first immobilized phase and a first liquid phase. The first immobilized phase is separated from the first liquid phase. An enzyme conjugate is prepared by coupling an enzyme and a second antibody which has a specific affinity for the antigen analysandum. The first immobilized phase containing the antigen analysandum bound to the immobilized antibody reactant is then reacted with a known quantity of the enzyme conjugate to form a second reaction mixture having a second immobilized phase and a second liquid phase. The second immobilized phase is then separated from the second liquid phase. The enzyme activity of the immobilized phase is directly proportional to the quantity of analysandum.

In an indirect sandwich assay, a reactant which is a first antigen to the antibody analysandum, and which is provided as a recombinant fusion protein, is immobilized in a solid phase. The analysandum is then reacted with an excess quantity of the immobilized reactant to form a first reaction mixture having a first immobilized phase and a first liquid phase. The first immobilized phase is separated from the first liquid phase. An enzyme conjugate is prepared by coupling an enzyme and a second binding protein, such as an antispecies antibody, having a specific binding affinity for the antibody analysandum. The first immobilized phase containing the antibody analysandum bound to the immobilized antigen reactant is then reacted with a known quantity of the enzyme conjugate to form a second reaction mixture having a second immobilized phase and a second liquid phase. The second immobilized phase is then separated from the second liquid phase. The enzyme activity of the immobilized phase is directly proportional to the quantity of analysandum. In a preferred embodiment, the enzyme linked immunosorbent assay is an indirect sandwich assay.

The known quantity of the enzyme-labeled reactant in the sandwich assay, prepared by coupling an enzyme and a second binding counterpart to the analysandum, is a predetermined quantity which will minimize nonspecific binding of the enzyme-labeled reactant to the solid phase (negative signal) and maximize the reaction of the enzyme-labeled reactant with the first immobilized phase containing the analysandum (positive signal). The assay range in an enzyme linked immunosorbent assay is very wide because the analysandum may be present in high or low amounts. Providing a large excess of enzyme-labeled reactant will result in reacting all analysandum with the enzyme-labeled reactant when the analysandum is present in high amounts but will result in significant nonspecific binding of the enzyme-labeled reactant to the solid phase when the analysandum is present in low amounts, thereby reducing the sensitivity of the assay. In general, accurate detection of all analysandum when the analysandum is present in low amounts is more important than detection of all analysandum when the analysandum is present in high amounts. Accordingly, the known quantity of the enzyme-labeled reactant is an intermediate quantity which will minimize nonspecific binding and maximize the reaction of the enzyme-labeled reactant with the analysandum. This known quantity of the enzyme-labeled reactant may be an excess quantity when the analysandum is present in low amounts and may not be an excess quantity when the analysandum is present in high amounts.

The second antibody which has a specific binding affinity for the antigen analysandum or the second binding protein which has a specific binding affinity for the antibody analysandum may be the same protein as the first binding counterpart in the sandwich assay or may be a third reactant which is a different protein. For example, the third reactant may be an antispecies antibody which is an antibody which reacts with an antibody from another species, such as a mouse antispecies antibody which reacts with a human antibody. In a preferred embodiment, the second specific binding protein in the indirect assay is a third reactant which is an antispecies antibody.

The present invention is further illustrated by the following examples which are not intended to limit the effective scope of the claims. All parts and percentages in the examples and throughout the specification and claims are by weight of the final composition unless otherwise specified.

## EXAMPLE

This example demonstrates the improved method of the present invention in an indirect sandwich enzyme linked immunosorbent assay. Specifically, the binding of a test sample (antibodies to hepatitis C virus) to the vehicular protein (superoxide dismutase) in a recombinant fusion protein was suppressed by premixing the test sample with the vehicular protein in denatured form.

Immobilized antigen reactant solutions or antigen coating solutions were made with the following recombinant superoxide dismutase (SOD) fusion proteins.

| C-100-3 | 0.5µg/ml-2µg/ml |
| C-22 | 0.4µg/ml-1µg/ml |
| C-200 | 1µg/ml-2µg/ml |

Each fusion protein, in the quantity set out above, was admixed with 0.05 M borate buffer solution (pH 9.0) containing 1.0 M of sodium chloride, sodium azide at 0.02%, phenol red at 0.00005%, and bovine serum albumin (BSA) at 10ug/ml.

The antigens C-100-3, C-22, and C-200 are recombinant superoxide dismutase fusion proteins for hepatitis C virus expressed in yeast. The fusion protein C-100-3 is described in published European patent application no. 388,232. The preparation and purification of fusion proteins C-22 and C-200 are carried out as described on pages 29-33 of published European patent application no. 388,232. With reference to FIGURE 17 of European patent application no. 388,232, the amino acid sequence of fusion protein C-100-3 is amino acids numbers 1569-1931, the amino acid sequence of fusion protein C-22 is amino acid numbers 2-120, and the amino acid sequence of fusion protein C-200 is amino acids numbers 1192-1930.

Immulon-1 microwells (sulfonated polystyrene, Dynatech, Chantilly, Virginia) were coated with 200ul of each of the antigen coating solutions set out above and left overnight at room temperature. The coating solutions were removed from the wells by aspiration and the wells were post coated with bovine serum albumin in phosphate buffered saline (PBS).

A sample diluent solution in phosphate buffered saline (pH 7.3) was prepared having the following components.

| | |
|---|---|
| Thimerosal | 0.02% |
| Sodium Chloride | 0.5 M |
| Denatured Superoxide Dismutase conveniently (100µg/ml-200µg/ml) preferably (100µg/ml) | 30ug/ml-300ug/ml |
| Triton X 100® | 1% |
| Yeast extract | 0.1%-1% |
| Casein | 0.1%-1% |
| Bovine serum albumin | 1%-3% |

The superoxide dismutase in the sample diluent solution was denatured by mixing a solution of SOD (70mg/ml) with a solution of urea (420mg/ml) and dithithreitol (1.54mg/ml). The resulting solution was then heated to about 60-65° C. for about two hours.

Wash buffer solution is available commercially under the tradename Ortho 20X Wash Buffer Concentrate from Ortho Diagnostics Systems Inc. Raritan, New Jersey. The enzyme conjugate solution, which is horseradish peroxidase conjugated to anti-human IpG (monoclonal) in phosphate buffered saline (pH 7.4) with stabilizing proteins, is available commercially from Ortho Diagnostics Systems Inc. Raritan, New Jersey. The enzyme substrate, which is ortho-phenylenediamine (OPD) in water with hydrogen peroxide, is available commercially from Ortho Diagnostics Systems Inc. Raritan, New Jersey as tablets mixed with substrate buffer (6ml).

The enzyme linked immunosorbent assay was carried out as follows. A quantity of 20µl of analysandum or sample (serum or plasma) was diluted in 200ul of sample diluent and added to an antigen coated microwell as set out above. The microwell was incubated for a period of about one hour at 37° C. The sample solution was then removed from the well by aspiration and the well was rinsed five times with wash buffer.

A quantity of 200µl of enzyme conjugate solution was added to the microwell and the well was incubated for a period of one hour at 37°C. The conjugate solution was then removed from the well by aspiration and the well was washed five times with wash buffer.

A quantity of 200µl of enzyme substrate solution was added to the microwell and the well was incubated for a period of 30 minutes at room temperature. A quantity of 50µl of 4N sulfuric acid solution was then added to the well and the optical density (OD) of the well was read at 490 NM. Reactivity was determined at a cutoff of 0.400 + mean of negative control optical density. Samples with an optical density at or above the cutoff were considered reactive for hepatitis C virus antibody.

Samples were also prepared containing no superoxide dismutase (control) and excess superoxide dismutase which had not been denatured (native SOD). The results of the assays are set out below in Table 1.

Table 1

| Sample | Optical Density (OD)$_{490}$ | | |
|---|---|---|---|
| | Control | Native SOD | Denatured SOD |
| 1 | 1.151 | 1.547 | 1.204 |
| 2* | 2.015 | 1.942 | 0.519 |
| 3 | 1.180 | 1.042 | 1.226 |
| 4* | 0.570 | 0.399 | 0.039 |
| 5 | 0.255 | 0.282 | 0.262 |

* Samples generating false positive signals when not treated with denatured superoxide dismutase.

Table 1   (continued)

| Sample | Optical Density (OD)$_{490}$ | | |
|---|---|---|---|
| | Control | Native SOD | Denatured SOD |
| 6 | 0.581 | 0.657 | 0.666 |
| 7 | 0.605 | 0.484 | 0.634 |
| 8 | 0.540 | 0.600 | 0.555 |
| 9 | 0.372 | 0.438 | 0.337 |
| 10* | 1.133 | 1.328 | 0.131 |
| 11* | 2.470 | 2.257 | 0.208 |
| 12 | 0.176 | 0.138 | 0.182 |
| 13 | 1.465 | 1.282 | 1.348 |
| 14 | 1.584 | 1.241 | 1.460 |
| 15 | 0.023 | 0.016 | 0.033 |
| 16 | 0.040 | 0.034 | 0.066 |
| 17 | 0.133 | 0.106 | 0.180 |

* Samples generating false positive signals when not treated with denatured superoxide dismutase.

Table 1 shows that samples 2, 4, 10, and 11 generated false positive signals when the test samples were not treated with denatured superoxide dismutase. The number of false positive signals, caused by reaction of the sample to the superoxide dismutase fusion protein, was suppressed in the samples containing denatured superoxide dismutase. The number of false positive signals was not suppressed in the control samples without added superoxide dismutase or the samples containing native superoxide dismutase. These examples show that the presence of denatured SOD in a test sample suppresses binding of the test sample to the vehicular protein SOD in the recombinant fusion protein and thereby improves assay specificity and maintains assay sensitivity.

## Claims

1. An enzyme linked immunosorbent assay for analyzing an analysandum which is a reactant in a reaction between binding counterparts wherein the counterparts comprise a bindable substance and a binding substance, which comprises the steps of:

    (a) providing a first reactant which is the binding counterpart of the analysandum as a recombinant fusion protein wherein the fusion protein comprises a first protein having the immunological reactivity of the first reactant and a second protein which is a vehicular protein fused to the first protein;
    (b) providing a second reactant which is an enzyme-labeled reactant wherein the enzyme-labeled reactant comprises a reactant which is the same as the analysandum coupled to an enzyme;
    (c) preparing a diluent solution of the analysandum by forming a solution of the analysandum with an effective amount of denatured vehicular protein to suppress binding of the analysandum to the vehicular protein in the recombinant fusion protein;
    (d) reacting the diluent solution of analysandum from step (c) with the recombinant fusion protein reactant from step (a) and the enzyme-labeled reactant from step (b) to form a reaction mixture, wherein the enzyme-labeled reactant is present in a known amount and the recombinant fusion protein reactant is present in a known amount insufficient to react with the total amount of analysandum and enzyme-labeled reactant; and
    (e) analyzing the enzyme activity of the reaction mixture which is directly proportional to the quantity of analysandum present in the reaction mixture.

2. The assay according to claim 1, wherein the bindable substance is an antigen selected from the group consisting of hepatitis C virus and human immunedeficiency virus.

3. The assay according to claim 1 or 2, wherein the vehicular protein in step (a) is selected from the group consisting of superoxide dismutase, *beta*-galactosidase, and lactamase.

4. The assay according to claim 3, wherein the vehicular protein in step (a) is superoxide dismutase.

5. An enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;
(b) immobilizing the recombinant fusion protein reactant from step (a) by contacting an aqueous solution of the recombinant fusion protein with a solid phase to immobilize the fusion protein on the solid phase;
(c) separating the aqueous solution from the solid phase in step (b) to prepare an immobilized reactant;
(d) providing a second reactant which is an enzyme-labeled reactant wherein the enzyme-labeled reactant comprises a reactant which is the same as the analysandum coupled to an enzyme;
(e) preparing a diluent solution of the analysandum by forming a solution of the analysandum with an effective amount of denatured vehicular protein to suppress binding of the analysandum to the vehicular protein in the recombinant fusion protein;
(f) reacting the diluent solution of analysandum from step (e) with the immobilized recombinant fusion protein reactant from step (c) and the enzyme-labeled reactant from step (d) to form a reaction mixture, wherein the enzyme-labeled reactant is present in a known amount and the recombinant fusion protein reactant is present in a known amount insufficient to react with the total amount of analysandum and enzyme-labeled reactant;
(g) separating the immobilized phase and the liquid phase; and
(h) analyzing the enzyme activity of the immobilized phase which is inversely proportional to the quantity of analysandum present in the reaction mixture.

6. The assay according to claim 5, wherein the bindable substance is an antigen selected from the group consisting of hepatitis C virus and human immunedeficiency virus.

7. The assay according to claim 5 or 6, wherein the vehicular protein in step (a) is selected from the group consisting of superoxide dismutase, *beta*-galactosidase, and lactamase.

8. The assay according to any one of claims 5 to 7, wherein the aqueous solution in step (b) has an ionic strength value from about 0.05 to about 1.0.

9. The assay according to any one of claims 5 to 8, wherein the solid phase is made of a polymer selected from the group consisting of polystyrene, sulfonated polystyrene, irradiated sulfonated polystyrene, and latex.

10. The assay according to any one of claims 5 to 9, further comprising the steps of (i) contacting the immobilized reactant in step (b) with an aqueous solution of a protein which is a nonreactant in the reaction to coat active sites on the solid phase and (ii) separating the aqueous solution from the immobilized reactant.

11. An enzyme linked immunosorbent assay for analyzing an analysandum which is a reactant in a reaction between binding counterparts wherein the counterparts comprise a bindable substance and a binding substance, which comprises the steps of:

(a) providing a first reactant which is the binding counterpart of the analysandum as a recombinant fusion protein wherein the fusion protein comprises a first protein having the immunological reactivity of the first reactant and a second protein which is a vehicular protein fused to the first protein;
(b) immobilizing the recombinant fusion protein reactant from step (a) by contacting an aqueous solution of the recombinant fusion protein with a solid phase to immobilize the fusion protein on the solid phase;
(c) separating the aqueous solution from the solid phase in step (b) to prepare an immobilized reactant;
(d) preparing a diluent solution of the analysandum by forming a solution of the analysandum with an effective amount of denatured vehicular protein to suppress binding of the analysandum to the vehicular protein in the recombinant fusion protein;
(e) reacting the diluent solution of analysandum from step (d) with the immobilized recombinant fusion protein reactant from step (c) to form a first reaction mixture having a first immobilized phase and a first liquid phase, wherein the immobilized reactant is present in an amount sufficient to react with all of the analysandum;
(f) separating the first immobilized phase and the first liquid phase in step (e);
(g) providing a second reactant which is an enzyme-labeled reactant wherein the enzyme-labeled reactant comprises a reactant which is a second binding counterpart to the analysandum coupled to an enzyme;

(h) reacting the enzyme-labeled reactant from step (g) with the first immobilized phase from step (f) to form a second reaction mixture having a second immobilized phase and a second liquid phase, wherein the enzyme-labeled reactant is present in a known amount;

(i) separating the second immobilized phase and the second liquid phase in step (h); and

(j) analyzing the enzyme activity of the second immobilized product phase in step (i) which is directly proportional to the quantity of analysandum present in the reaction mixture.

12. The assay according to claim 11, wherein the analysandum is a binding substance selected from the group consisting of an antibody and a specific binding protein.

13. The assay according to claim 12, wherein the binding substance is an antibody selected from the group consisting of hepatitis C virus antibody and human immunedeficiency virus antibody.

14. The assay according to claim 11, wherein the analysandum is a bindable substance selected from the group consisting of an antigen and a hapten.

15. The assay according to any one of claims 11 to 14, wherein the vehicular protein in step (a) is selected from the group consisting of superoxide dismutase, *beta*-galactosidase, and lactamase.

16. The assay according to any one of claims 11 to 15, wherein the aqueous solution in step (b) has an ionic strength value from about 0.05 to about 1.

17. The assay according to any one of claims 11 to 16, wherein the solid phase is made of a polymer selected from the group consisting of polystyrene, sulfonated polystyrene, irradiated sulfonated polystyrene, and latex.

18. The assay according to any one of claims 11 to 17, further comprising the steps of (i) contacting the immobilized reactant in step (b) with an aqueous solution of a protein which is a nonreactant in the reaction to coat active sites on the solid phase and (ii) separating the aqueous solution from the immobilized reactant.

19. The assay according to any one of claims 11 to 18, wherein the second binding counterpart to the analysandum in step (g) is a third reactant different from the reactant in step (a) and is an antispecies antibody.

20. The assay according to any one of claims 11 to 18, wherein the second binding counterpart to the analysandum in step (g) is the same as the reactant in step (a).

## Patentansprüche

1. Enzymverbundener Immunadsorptionstest zur Analyse eines Analysanden, der ein Reaktionspartner in einer Reaktion zwischen bindenden Gegenstücken ist, wobei die Gegenstücke eine bindungsfähige Substanz und eine bindende Substanz umfassen, umfassend die Schritte:

(a) Bereitstellung eines ersten Reaktionspartners, der das bindende Gegenstück des Analysanden ist, als ein rekombinantes Fusionsprotein, wobei das Fusionsprotein ein erstes Protein umfaßt, das die immunologische Reaktivität des ersten Reaktionspartners besitzt, und ein zweites Protein, das ein an das erste Protein fusioniertes Trägerprotein ist;

(b) Bereitstellung eines zweiten Reaktionspartners, der ein enzymmarkierter Reaktionspartner ist, wobei der enzymmarkierte Reaktionspartner einen Reaktionspartner umfaßt, der der gleiche ist wie der an ein Enzym gekoppelte Analysand;

(c) Herstellung einer Verdünnungslösung des Analysanden durch die Erzeugung einer Lösung des Analysanden mit einer wirksamen Menge von denaturiertem Trägerprotein, um die Bindung des Analysanden an das Trägerprotein in dem rekombinanten Fusionsprotein zu unterbinden;

(d) Umsetzung der Verdünnungslösung des Analysanden aus Schritt (c) mit dem rekombinanten Fusionsproteinreaktionspartner aus Schritt (a) und dem enzymmarkierten Reaktionspartner aus Schritt (b) zur Erzeugung eines Reaktionsgemisches, wobei der enzymmarkierte Reaktionspartner in einer bekannten Menge vorhanden ist und der rekombinante Fusionsprotein-Reaktionspartner in einer bekannten Menge vorhanden ist, die nicht ausreicht, um mit der gesamten Menge des Analysanden und des enzymmarkierten Reaktionspartner zu reagieren; und

(e) Analyse der Enzymaktivität des Reaktionsgemisches, die direkt proportional zu der Menge des Analysanden im Reaktionsgemisch ist.

2. Test nach Anspruch 1, wobei die bindungsfähige Substanz ein Antigen aus der Gruppe Hepatitis C-Virus und menschliche Immunschwäche-Virus ist.

3. Test nach Anspruch 1 oder 2, wobei das Trägerprotein in Schritt (a) ausgewählt ist aus Superoxiddismutase, β-Galactosidase und Lactamase.

4. Test nach Anspruch 3, wobei das Trägerprotein in Schritt (a) Superoxiddismutase ist.

5. Enzymverbundener Immunadsorptionstest zur Analyse eines Analysanden, der ein Reaktionspartner in einer Reaktion zwischen bindenden Gegenstücken ist, wobei die Gegenstücke eine bindungsfähige Substanz und eine bindende Substanz umfassen, umfassend die Schritte:

(a) Bereitstellung eines ersten Reaktionspartners, der das bindende Gegenstück des Analysanden ist, als ein rekombinantes Fusionsprotein, wobei das Fusionsprotein ein erstes Protein umfaßt, das die immunologische Reaktivität des ersten Reaktionspartners besitzt, und ein zweites Protein, das ein an das erste Protein fusioniertes Trägerprotein ist,
(b) Immobilisierung des rekombinanten Fusionsprotein-Reaktionspartners aus Schritt (a) durch Inkontaktbringen einer wäßrigen Lösung des rekombinanten Fusionsproteins mit einer festen Phase, um das Fusionsprotein an einer festen Phase zu immobilisieren;
(c) Trennung der wäßrigen Phase von der festen Phase aus Schritt (b) zur Herstellung eines immobilisierten Reaktionspartners;
(d) Bereitstellung eines zweiten Reaktionspartners, der ein enzymmarkierter Reaktionspartner ist, wobei der enzymmarkierte Reaktionspartner einen Reaktionspartner umfaßt, der der gleiche ist wie der an ein Enzym gekoppelte Analysand;
(e) Herstellung einer Verdünnungslösung des Analysanden durch Erzeugung einer Lösung des Analysanden mit einer wirksamen Menge von denaturiertem Trägerprotein, um die Bindung des Analysanden an das Trägerprotein in dem rekombinanten Fusionsprotein zu unterbinden;
(f) Umsetzung der Verdünnungslösung des Analysanden aus Schritt (e) mit dem immobilisierten rekombinanten Fusionsprotein-Reaktionspartners aus Schritt (c) und dem enzymmarkierten Reaktionspartner aus Schritt (d) zur Erzeugung eines Reaktionsgemisches, wobei der enzymmarkierte Reaktionspartner in einer bekannten Menge vorhanden ist, und der rekombinante Fusionsprotein-Reaktionspartner in einer bekannten Menge vorhanden ist, die nicht ausreicht, um mit der gesamten Menge des Analysanden und des enzymmarkierten Reaktionspartners zu reagieren;
(g) Trennung der immobilisierten Phase und der flüssigen Phase; und
(h) Analyse der Enzymaktivität der immobilisierten Phase, die umgekehrt proportional zu der Menge des Analysanden in dem Reaktionsgemisch ist.

6. Test nach Anspruch 5, wobei die bindungsfähige Substanz ein Antigen aus der Gruppe Hepatitis C-Virus und menschliche Immunschwäche-Virus ist.

7. Test nach Anspruch 5 oder 6, wobei das Trägerprotein in Schritt (a) ausgewählt ist aus Superoxiddismutase, β-Galactosidase und Lactamase.

8. Test nach einem der Ansprüche 5 bis 7, wobei die wäßrige Lösung in Schritt (b) eine Ionenstärke von etwa 0,05 bis etwa 1,0 hat.

9. Test nach einem der Ansprüche 5 bis 8, wobei die feste Phase aus einem Polymer gemacht ist, ausgewählt aus Polystyrol, sulfoniertem Polystyrol, bestrahltem sulfoniertem Polystyrol und Latex.

10. Test nach einem der Ansprüche 5 bis 9, weiter umfassend die Schritte (i) Inkontaktbringen des immobilisierten Reaktionspartners in Schritt (b) mit einer wäßrigen Lösung eines Proteins, das ein Nicht-Reaktionspartner der Umsetzung ist, um die aktiven Stellen der festen Phase abzudecken, und (ii) Trennung der wäßrigen Lösung und des immobilisierten Reaktionspartners.

11. Enzymverbundener Immunadsorptionstest zur Analyse eines Analysanden, der ein Reaktionspartner in einer Reaktion zwischen bindenden Gegenstücken ist, wobei die Gegenstücke eine bindungsfähige Substanz und eine bindende Substanz umfassen, umfassend die Schritte:

(a) Bereitstellung eines ersten Reaktionspartners, der das bindende Gegenstück des Analysanden ist, als ein rekombinantes Fusionsprotein, wobei das Fusionsprotein ein erstes Protein umfaßt, das die immunologische Reaktivität des ersten Reaktionspartners besitzt, und ein zweites Protein, das ein an das erste Protein fusioniertes Trägerprotein ist;

(b) Immobilisierung des rekombinanten Fusionsprotein-Reaktionspartners aus Schritt (a) durch Inkontaktbringen einer wäßrigen Lösung des rekombinanten Fusionsproteins mit einer festen Phase, um das Fusionsprotein an die feste Phase zu immobilisieren;

(c) Trennung der wäßrigen Lösung von der festen Phase aus Schritt (b) zur Herstellung eines immobilisierten Reaktionspartners;

(d) Herstellung einer Verdünnungslösung des Analysanden durch Erzeugen einer Lösung des Analysanden mit einer wirksamen Menge von denaturiertem Trägerprotein, um die Bindung des Analysanden an das Trägerprotein in dem rekombinanten Fusionsprotein zu unterbinden;

(e) Umsetzung der Verdünnungslösung des Analysanden aus Schritt (d) mit dem immobilisierten rekombinanten Fusionsprotein-Reaktionspartner aus Schritt (c) zur Erzeugung eines ersten Reaktionsgemisches, das eine erste immobilisierte Phase und eine erste flüssige Phase besitzt, wobei der immobilisierte Reaktionspartner in einer Menge vorhanden ist, die ausreicht, um mit dem gesamten Analysanden zu reagieren;

(f) Trennung der ersten immobilisierten Phase und der ersten flüssigen Phase aus Schritt (e);

(g) Bereitstellung eines zweiten Reaktionspartners, der ein enzymmarkierter Reaktionspartner ist, wobei der enzymmarkierte Reaktionspartner einen Reaktionspartner umfaßt, der ein zweites bindendes Gegenstück des Analysanden ist, gekoppelt an ein Enzym;

(h) Umsetzung des enzymmarkierten Reaktionspartners aus Schritt (g) mit der ersten immobilisierten Phase aus Schritt (f) zur Erzeugung eines zweiten Reaktionsgemisches, das eine zweite immobilisierte Phase und eine zweite flüssige Phase besitzt; wobei der enzymmarkierte Reaktionspartner in einer bekannten Menge vorhanden ist;

(i) Trennung der zweiten immobilisierten Phase und der zweiten flüssigen Phase aus Schritt (h); und

(j) Analyse der Enzymaktivität der zweiten hergestellten immobilisierten Phase in Schritt (i), die direkt proportional zu der Menge des Analysanden im Reaktionsgemisch ist.

12. Test nach Anspruch 11, wobei der Analysand eine bindende Substanz ist, ausgewählt aus einem Antikörper und einem spezifischen Bindungsprotein.

13. Test nach Anspruch 12, wobei die bindende Substanz ein Antikörper ist, ausgewählt aus einem Hepatitis C-Virus-Antikörper und einem menschlichen Immunschwäche-Virus-Antikörper.

14. Test nach Anspruch 11, wobei der Analysand eine bindungsfähige Substanz ist, ausgewählt aus einem Antigen und einem Hapten.

15. Test nach einem der Ansprüche 11 bis 14, wobei das Trägerprotein in Schritt (a) ausgewählt ist aus Superoxiddismutase, β-Galactosidase und Lactamase.

16. Test nach einem der Ansprüche 11 bis 15, wobei die wäßrige Lösung in Schritt (b) eine Ionenstärke im Wert von etwa 0,05 bis etwa 1 hat.

17. Test nach einem der Ansprüche 11 bis 16, wobei die feste Phase aus einem Polymer gemacht ist, ausgewählt aus Polystyrol, sulfoniertem Polystyrol, bestrahltem sulfoniertem Polystyrol und Latex.

18. Test nach einem der Ansprüche 11 bis 17, weiter umfassend die Schritte (i) Inkontaktbringen des immobilisierten Reaktionspartners in Schritt (b) mit einer wäßrigen Lösung eines Proteins, das ein Nicht-Reaktionspartner in der Reaktion ist, um die aktiven Stellen der festen Phase abzudecken, und (ii) Trennung der wäßrigen Lösung von dem immobilisierten Reaktionspartner.

19. Test nach einem der Ansprüche 11 bis 18, wobei das zweite bindende Gegenstück zu dem Analysanden in Schritt (g) ein dritter Reaktionspartner ist, der verschieden von dem Reaktionspartner in Schritt (a) und ein Antispecies-Antikörper ist.

**20.** Test nach einem der Ansprüche 11 bis 18, wobei das zweite bindende Gegenstück zu dem Analysanden in Schritt (g) der gleiche wie der Reaktionspartner in Schritt (a) ist.

**Revendications**

1. Dosage par immunosorption à liaison enzymatique (E.L.I.S.A.) pour analyser un analysandum qui est un réactif dans une réaction entre des contreparties de liaison dans lequel les contreparties comprennent une substance susceptible de se lier et d'une substance liante, qui comprend les étapes consistant à :

   (a) se munir d'un premier réactif qui est la contrepartie de liaison de l'analysandum en tant que protéine de fusion recombinante, dans lequel la protéine de fusion comprend une première protéine ayant la réactivité immunologique du premier réactif et une seconde protéine qui est une protéine vectrice fusionnée à la première protéine ;
   (b) se munir d'un second réactif qui est un réactif marqué par une enzyme dans lequel le réactif marqué par une enzyme comprend un réactif qui est le même que l'analysandum couplé à une enzyme ;
   (c) préparer une solution diluée de l'analysandum en formant une solution de l'analysandum avec une quantité efficace de protéine vectrice dénaturée pour supprimer la liaison de l'analysandum à la protéine vectrice dans la protéine de fusion recombinante ;
   (d) faire réagir la solution diluée d'analysandum de l'étape (c) avec le réactif constitué par la protéine de fusion recombinante de l'étape (a) et le réactif marqué par une enzyme de l'étape (b) pour former un mélange réactionnel, dans lequel le réactif marqué par une enzyme est présent en quantité connue et le réactif constitué par la protéine de fusion recombinante est présent en quantité connue, insuffisante pour réagir avec la quantité totale d'analysandum et de réactif marqué par une enzyme ; et
   (e) analyser l'activité enzymatique du mélange réactionnel qui est directement proportionnelle à la quantité d'analysandum présente dans le mélange réactionnel.

2. Dosage selon la revendication 1, dans lequel la substance susceptible de se lier est un antigène choisi dans le groupe constitué par le virus de l'hépatite C et le virus HIV.

3. Dosage selon la revendication 1 ou 2, dans lequel la protéine vectrice dans l'étape (a) est choisie dans le groupe constitué par la superoxyde dismutase, la β-galactosidase et la lactamase.

4. Dosage selon la revendication 3, dans lequel la protéine vectrice dans l'étape (a) est la superoxyde dismutase.

5. Dosage par immunosorption à liaison enzymatique (E.L.I.S.A.) pour analyser un analysandum qui est un réactif dans une réaction entre des contreparties de liaison dans lequel les contreparties comprennent une substance susceptible de se lier et une substance liante, qui comprend les étapes consistant à:

   (a) se munir d'un premier réactif qui est la contrepartie de liaison de l'analysandum en tant que protéine de fusion recombinante, dans lequel la protéine de fusion comprend une première protéine ayant la réactivité immunologique du premier réactif et une seconde protéine qui est une protéine vectrice fusionnée à la première protéine ;
   (b) immobiliser le réactif constitué de la protéine de fusion recombinante de l'étape (a) en mettant en contact une solution aqueuse de la protéine de fusion recombinante avec une phase solide pour immobiliser la protéine de fusion sur la phase solide ;
   (c) séparer la solution aqueuse de la phase solide dans l'étape (b) pour préparer un réactif immobilisé ;
   (d) se munir d'un second réactif qui est un réactif marqué par une enzyme dans lequel le réactif marqué par une enzyme comprend un réactif qui est le même que l'analysandum couplé à une enzyme ;
   (e) préparer une solution dans un diluant de l'analysandum en formant une solution de l'analysandum avec une quantité efficace de protéine vectrice dénaturée pour supprimer la liaison de l'analysandum à la protéine vectrice dans la protéine de fusion recombinante ;
   (f) faire réagir la solution dans le diluant d'analysandum de l'étape (e) avec le réactif constitué par la protéine de fusion recombinante immobilisée de l'étape (c) et le réactif marqué par une enzyme de l'étape (d) pour former un mélange réactionnel, dans lequel le réactif marqué par une enzyme est présent en quantité connue et le réactif constitué par la protéine de fusion recombinante est présent en quantité connue, insuffisante pour réagir avec la quantité totale d'analysandum et de réactif marqué par une enzyme ;
   (g) séparer la phase immobilisée et la phase liquide ; et

(h) analyser l'activité enzymatique de la phase immobilisée qui est inversement proportionnelle à la quantité d'analysandum présente dans le mélange réactionnel.

6. Dosage selon la revendication 5, dans lequel la substance susceptible de se lier est un antigène choisi dans le groupe constitué par le virus de l'hépatite C et le virus HIV.

7. Dosage selon la revendication 5 ou 6, dans lequel la protéine vectrice dans l'étape (a) est choisie dans le groupe constitué par la superoxyde dismutase, la β-galactosidase et la lactamase.

8. Dosage selon l'une quelconque des revendications 5 à 7, dans lequel la solution aqueuse dans l'étape (b) possède une force ionique d'environ 0,05 à environ 1,0.

9. Dosage selon l'une quelconque des revendications 5 à 8, dans lequel la phase solide est faite d'un polymère choisi dans le groupe constitué par le polystyrène, le polystyrène sulfoné, le polystyrène sulfoné irradié et le latex.

10. Dosage selon l'une quelconque des revendications 5 à 9, comprenant par ailleurs les étapes consistant à (i) mettre en contact le réactif immobilisé dans l'étape (b) avec une solution aqueuse d'une protéine qui est non réactive dans la réaction pour enrober les sites actifs sur la phase solide et (ii) séparer la solution aqueuse du réactif immobilisé.

11. Dosage par immunosorption à liaison enzymatique (E.L.I.S.A.) pour analyser un analysandum qui est un réactif dans une réaction entre des contreparties de liaison dans lequel les contreparties comprennent une substance susceptible de se lier et une substance liante, qui comprend les étapes consistant à :

(a) se munir d'un premier réactif qui est la contrepartie de liaison de l'analysandum en tant que protéine de fusion recombinante, dans lequel la protéine de fusion comprend une première protéine ayant la réactivité immunologique du premier réactif et une seconde protéine qui est une protéine vectrice fusionnée à la première protéine ;
(b) immobiliser le réactif constitué de la protéine de fusion recombinante de l'étape (a) en mettant en contact une solution aqueuse de la protéine de fusion recombinante avec une phase solide pour immobiliser la protéine de fusion sur la phase solide ;
(c) séparer la solution aqueuse de la phase solide dans l'étape (b) pour préparer un réactif immobilisé ;
(d) préparer une solution diluée de l'analysandum en formant une solution de l'analysandum avec une quantité efficace de protéine vectrice dénaturée pour supprimer la liaison de l'analysandum à la protéine vectrice dans la protéine de fusion recombinante ;
(e) faire réagir la solution diluée d'analysandum de l'étape (d) avec le réactif constitué par la protéine de fusion recombinante immobilisée de l'étape (c) pour former un premier mélange réactionnel comportant une première phase immobilisée et une première phase liquide, dans lequel le réactif immobilisé est présent en quantité suffisante pour réagir avec la totalité de l'analysandum ;
(f) séparer la première phase immobilisée et la première phase liquide dans l'étape (e);
(g) se munir d'un second réactif qui est un réactif marqué par une enzyme dans lequel le réactif marqué par une enzyme comprend un réactif qui est une seconde contrepartie de liaison de l'analysandum couplé à une enzyme ;
(h) faire réagir le réactif marqué par une enzyme de l'étape (g) avec la première phase immobilisée de l'étape (f) pour former un second mélange réactionnel comportant une seconde phase immobilisée et une seconde phase liquide, dans lequel le réactif marqué par une enzyme est présent en quantité connue ;
(i) séparer la seconde phase immobilisée et la seconde phase liquide dans l'étape (h) ; et
(j) analyser l'activité enzymatique de la seconde phase de produit immobilisée dans l'étape (i), qui est directement proportionnelle à la quantité d'analysandum présente dans le mélange réactionnel.

12. Dosage selon la revendication 11, dans lequel l'analysandum est une substance liante choisie dans le groupe constitué par un anticorps et une protéine liante spécifique.

13. Dosage selon la revendication 12, dans lequel la substance liante est un anticorps choisi dans le groupe constitué par l'anticorps du virus de l'hépatite C et l'anticorps du virus HIV.

14. Dosage selon la revendication 11, dans lequel l'analysandum est une substance susceptible de se lier choisie dans le groupe constitué par un antigène et un haptène.

**15.** Dosage selon l'une quelconque des revendications 11 à 14, dans lequel la protéine vectrice dans l'étape (a) est choisie dans le groupe constitué par la superoxyde dismutase, la β-galactosidase et la lactamase.

**16.** Dosage selon l'une quelconque des revendications 11 à 15, dans lequel la solution aqueuse dans l'étape (b) possède une force ionique d'environ 0,05 à environ 1.

**17.** Dosage selon l'une quelconque des revendications 11 à 16, dans lequel la phase solide est faite d'un polymère choisi dans le groupe constitué par le polystyrène, le polystyrène sulfoné, le polystyrène sulfoné irradié et le latex.

**18.** Dosage selon l'une quelconque des revendications 11 à 17, comprenant par ailleurs les étapes consistant à (i) mettre en contact le réactif immobilisé dans l'étape (b) avec une solution aqueuse d'une protéine qui est non réactive dans la réaction pour enrober les sites actifs sur la phase solide et (ii) séparer la solution aqueuse du réactif immobilisé.

**19.** Dosage selon l'une quelconque des revendications 11 à 18, dans lequel la seconde contrepartie de liaison de l'analysandum dans l'étape (g) est un troisième réactif différent du réactif dans l'étape (a) et est un anticorps anti-espèce.

**20.** Dosage selon l'une quelconque des revendications 11 à 18, dans lequel la seconde contrepartie de liaison de l'analysandum dans l'étape (g) est la même que le réactif dans l'étape (a).